# EUROPEAN PATENT SPECIFICATION

(11) **EP 0 565 812 B1**
(45) Date of publication and mention of the grant of the patent: **14.05.1997**
(21) Application number: 93100439.4
(22) Date of filing: 14.01.1993
(51) Int. Cl.: A61B 17/72

(54) **Humerus nail**
Humerusnagel
Clou huméral

(30) Priority: 11.04.1992 DE 9205099 U
(43) Date of publication of application: 20.10.1993
(73) Proprietor: Howmedica GmbH, D-24232 Schönkirchen (DE)
(72) Inventor: Harder, Hans Erich, W-2316 Probsteierhagen (DE)
(74) Representative: Patentanwälte Hauck, Graalfs, Wehnert, Döring, Siemons

(56) References cited:
- EP-A- 0 226 701
- WO-A-89/07056
- DE-U- 9 101 035
- DE-U- 9 102 018
- FR-A- 2 387 637
- US-A- 4 622 959
- US-A- 4 827 917

## Description

The present invention relates to a humerus nail according to the preamble of the claim 1.

A humerus nail of this type is disclosed in DE-GM 91 01 035.7 The proximal shaft portion of the prior nail is angled with respect to the longitudinal axis of the shaft including a pair of cross bores extending through the proximal shaft portion peripherally with respect to offset each other. Due to its angled shape, there is substantially a single point of impact only depending on the anatomic conditions. The limited point of impact in driving the nail into the medullary canal further determines the position of the cross bores and thus the position of the screws lateral to be inserted,provoking the danger, for example, that nerves associated with the proximal humerus region may be hurt in anchoring the nail using the cross bores. This danger is further increased by the screws inserted extending in different directions from the humerus with respect to the pheriphery thereof.

From the DE-U-91 02 018 it has become known to provide a medullary nail with a pair of cross bores in the proximal portion. The shaft of the nail is straight throughout its length and proximally inserted into the medullary cavity. However, it can be derived from this reference that it must be applied to fractures of tibia or femur.

The present invention is based on the object to provide a humerus nail which avoids the drawbacks referred to and which, in particular, reduces the danger to hurt nerves in anchoring the nail.

The object referred to is solved by the features of claim 1.

The humerus nail according to the invention comprises a shaft to be proximally inserted into the medularry canal of the humerus, wherein the entire length of the shaft is straight, the shaft including a pair of cross bores in the proximal region thereof. Furthermore, axis parallel slots are provided at the distal shaft end. Due to the straight extension of the shaft, the humerus nail may be proximally inserted into the medularry canal of the humerus in an appropriate position, wherein the position of the cross bores is not yet fixed since the straight shaft can be rotated about the longitudinal axis after being inserted. The appropriate position of the cross bore is selected such that the screw to be inserted into the cross bore in anchoring the nail cannot hurt a nerv path. The position of the cross bore may be detected by a target means of known structure.

The humerus nail according to an embodiment of the invention includes a screw to be distally inserted into the shaft which screw includes a threaded portion cooperating with an internal thread in the shaft and an expanding body at the head portion to expand the slotted distal end of the shaft when srewed in. A screw of this type including an expanding body is disclosed in DE-GM 91 01 035.7.

According to an embodiment of the invention the shaft includes a pair of cross bores in the proximal region thereof which cross bores can extend parallel with respect to each other. Accordingly the screws inserted into the cross bores project from the nail at the same side so that there is no danger of hurting adjacent nerves by the screws after the nail has been fixed in an appropriate position.

The cross bores extend under an angle of approximately 55 degrees with respect to the longitudinal axis of the shaft, wherein at least a cross bore can include an internal thread or, respectively, a semi finished internal thread. The incomplete internal thread can be provided for the cross bore facing the distal end of the shaft, for example.

This prevents, that the screw inserted into the cross bore including the semi-thread pierces through the nail. This offers a further chance to check the correct position of the cross bore to prevent a damage to the nerves and to fix the bone fragments. Subsequently, the screw and a further screw can be inserted through the cross bores in anchoring the nail. The distal end of the humerus nail can be fixed to the bone by the screw with the expanding body which is inserted into the shaft from the distal end in a known manner.

An embodiment of the present invention is described in detail in referring to the drawings as follows.
- Fig. 1: is a side view of a humerus nail according to the invention, partly in section.
- Fig. 2: is a longitudinal section in a larger scale through the distal end of the humerus nail.
- Fig. 3: shows the detail of Fig. 1 in an enlarged scale and
- Fig. 4: is a cross section in a larger scale through a humerus nail along the line A-A of Fig. 1.

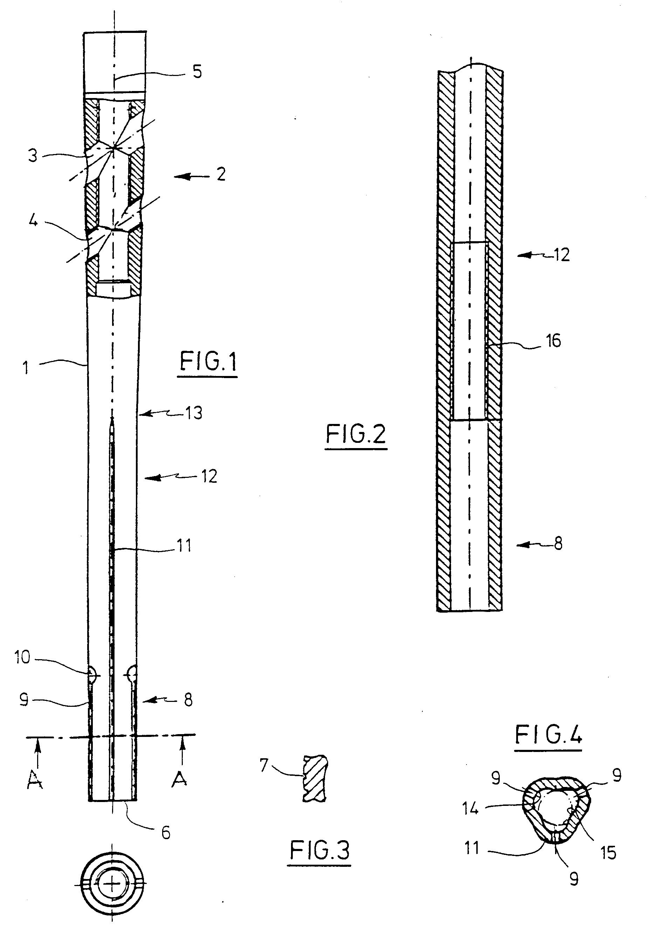

The humerus nail shown in Fig. 1 comprises a straight shaft 1 having a proximal portion 2 in which a pair of cross bores 3, 4 is provided to receive screws (not shown) for anchoring the nail in the bone. The cross bores 3, 4 extend parallel with respect to each other and extend under an angle of 55 degrees with respect to the longitudinal axis 5 of the shaft 1. The cross bore 4 facing the distal end 6 of the shaft includes an incomplete internal thread 7 (Fig. 3).

In the distal portion 8 of the shaft axis, parallel slots 9 extend from the distal end 6 up to the enlargement 10. Three raised portions 11 are provided via the periphery of the humerus nail which raised portions merge at 13 into the intermediate portion 12 of the shaft 1.

According to Fig. 4 the raised portions 11 at the outer periphery correspond to indentations 14 at the inner periphery of the shaft 1. Between the indentations 14, the inner periphery is provided with raised portions 15. The inner periphery of the shaft 1 limited by the raised portions 15 tapers from the distal end 6 towards the proximal portion 2 of the shaft.

The raised portions 15 are provided with an internal thread 16 according to Fig. 2.

The straight humerus nail is inserted from proximal into the medullary canal of the humerus. Thereafter the nail can be rotated about its longitudinal axis to obtain an appropriate position of the cross bores to insert the screws. This makes sure that no nerves are hurt in anchoring the nail in the bone by the lateral screws. The semi-profiled thread 7 of the cross bore 4 initially prevents that a lateral screw inserted into the bore 4 pierces through the nail. After a further possible check of the position of the cross bore, the lateral screw is inserted through the nail and the bone. Thereafter a second screw can be inserted into the cross bore 3. The position of the cross bores can be detected by a known target device.

A known screw (not shown) is inserted from the distal end 6 of the shaft 1. The screw comprises a threaded portion cooperating with the inner thread 16 of the shaft 1 and an expanding member at the head thereof for expanding the distal portion 8 of the shaft including the axis-parallel slots 9. Thus the humerus nail is also distally anchored in the bone.

## Claims

1. A humerus nail comprising a shaft (1) to be proximally inserted into the medullary cavity of the humerus, where the shaft
- in its entire length is straight
- includes a pair of cross bores (3, 4) in the proximal portion thereof which
- extend parallel to each other
characterised in that the cross bores extend
- under an angle of approximately 55° relative to the longitudinal axes (5) of the shaft (1)
- with at least that cross bore facing the distal end (6) of the shaft being provided with an internal thread, or respectively a semi-profile inner thread (7)
- said shaft having an inner thread (16) near its intermediate portion (12) and a distal portion (8) which is slotted for the accommodation of a screw with an expanding body to expand the distal portion (8).

## Patentansprüche

1. Humerusnagel mit einem von proximal in die Markhöhe des Humerus einführbaren Schaft (1), bei dem der Schaft
- über seine gesamte Länge gerade ist
- in seinem proximalen Abschnitt ein Paar von Querbohrungen (3, 4) aufweist, die
- parallel zueinander verlaufen
dadurch gekennzeichnet, daß die Querbohrungen
- unter einem Winkel von etwa 55° gegenüber der Längsachse (5) des Schaftes (1) sich erstrecken,
- wobei zumindest diese Bohrung dem distalen Ende (6) des Schaftes zugekehrt und mit einem Innengewinde versehen ist bzw. einem angedeuteten Innengewinde (7),
- wobei der Schaft ein Innengewinde (16) nahe seinem mittleren Abschnitt (12) aufweist und ein distaler Abschnitt (8) geschlitzt ist für die Aufnahme einer Schraube mit einem Spreizkörper, um den distalen Abschnitt (8) zu spreizen.

## Revendications

1. Clou huméral comportant une tige (1) destinée à être introduite de façon proximale dans la cavité médullaire de l'humérus, où la tige
- est droite sur toute la longueur,
- présente deux lumières croisées (3, 4) dans sa partie proximale, lesquelles
- s'étendent parallèlement l'une à l'autre,
caractérisé en ce que les lumières croisées s'étendent
- sous un angle d'environ 55° par rapport à l'axe longituidnal (5) de la tige (1),
- avec au moins la lumière transversale faisant face à l'extrémité distale (6) de la tige pourvue d'un filet intérieur, ou bien, respectivement, d'un filet intérieur (7) à demi-profil,
- ladite tige ayant un filet intérieur (16) proche de sa partie intermédiaire (12) et une partie distale (8) qui est fendue pour recevoir une vis ayant un corps expansible pour expanser la partie distale (8).
